# EUROPÄISCHE PATENTSCHRIFT

(11) **EP 4 025 297 B1**
(45) Veröffentlichungstag und Bekanntmachung des Hinweises auf die Patenterteilung: **08.07.2026**
(21) Anmeldenummer: 20757266.0
(22) Anmeldetag: 12.08.2020
(51) Int. Cl.: A61N 5/06

(54) **VORRICHTUNG ZUR UV-ANTISEPTIK**
APPARATUS FOR UV STERILIZATION
DISPOSITIF D'ANTISEPSIE PAR RAYONNEMENT ULTRAVIOLET

(30) Priorität: 06.09.2019 DE 102019124017
(43) Veröffentlichungstag der Anmeldung: 13.07.2022
(73) Patentinhaber: Universitätsmedizin Greifswald, 17457 Greifswald (DE); Charité - Universitätsmedizin Berlin Körperschaft des öffentlichen Rechts, 10117 Berlin (DE); Technische Universität Berlin, 10623 Berlin (DE); Ferdinand-Braun-Institut gGmbH, Leibniz-Institut für Höchstfrequenztechnik, 12489 Berlin (DE)
(72) Erfinder: MEINKE, Martina, 14193 Berlin (DE); LADEMANN, Jürgen, 15517 Fürstenwalde (DE); KRAMER, Axel, 17489 Greifswald (DE); KNEISSL, Michael, 10435 Berlin (DE); WERNICKE, Tim, 10635 Berlin (DE); WINTERWERBER, Ulrike, 12357 Berlin (DE); EINFELDT, Sven, 12623 Berlin (DE)
(74) Vertreter: Gulde & Partner
(86) Internationale Anmeldenummer: PCT/EP2020/072631
(87) Internationale Veröffentlichungsnummer: WO 2021/043554

(56) Entgegenhaltungen:
- EP-A1- 3 195 900
- US-A1- 2006 167 531
- US-A1- 2016 303 394

## Beschreibung

Die Erfindung betrifft eine Vorrichtung und ein Verfahren zur UV-Antiseptik, insbesondere zur intrakorporalen in-vivo UV-Antiseptik am menschlichen und tierischen Körper bei einer Besiedlung mit multiresistenten Erregern (MRE) wie Methicillin-resistenten Staphylococcus aureus (MRSA) und Staphylococcus epidermidis (MRSE).

Die pandemische Ausbreitung multiresistenter Erreger (MRE) führt weltweit vermehrt zu Infektionen mit einhergehender erhöhter Sterblichkeit insbesondere bei Intensivpatienten. Bestehende Dekolonisierungskonzepte sind nur unzureichend erfolgreich. Die Dekolonisation von MRE erfolgt derzeit überwiegend mittels Antiseptik. Die Erfolgsquote liegt dabei bei jedoch weit unterhalb von 50 %. Wesentliche Regionen des Körpers sind zudem über Antiseptik nicht zugänglich.

Für die UV-Antiseptik (auch als UV-Sterilisation oder UV-Desinfektion bezeichnet) von Oberflächen bzw. medizinischem Gerät werden typischerweise UV-Quecksilberdampflampen oder UV-LEDs eingesetzt, welche bei 254 nm bzw. im Wellenlängenbereich von 250 nm bis 285 nm emittieren. Die Auswahl der Wellenlänge ergibt sich aus dem Umstand, dass für diesen Wellenlängenbereich die Wirkung der UV-Strahlung ("Germicidal Effectiveness") ihr Maximum aufweist. Es handelt sich um Strahlung insbesondere aus dem UVC-Bereich, welchem definitionsgemäß Wellenlängen zwischen 100 nm und 280 nm zugeordnet werden. Bei der UV-Antiseptik führt die zugeführte Strahlung zu einer irreversiblen Schädigung der genetischen Erbinformation von Bakterien und Viren, wodurch deren Metabolismus zusammenbricht. Eine Anwendung der UVC-Strahlung ist auch am Menschen möglich (z.B. zur Antiseptik von Erregern, insbesondere MRSA und MRSE), birgt allerdings das Problem, das die UVC-Strahlung in diesen Wellenlängenbereichen tief in die Hautschichten eindringt und zu einer entsprechenden Schädigung auch des gesunden menschlichen Gewebes führt.

Aus kürzlich veröffentlichten Studien ist bekannt, dass insbesondere kurzwellige Strahlung mit Wellenlängen ≤ 230 nm aufgrund einer hohen Absorption in den oberen Hautschichten nicht tief in die Haut, Schleimhaut oder Wunde eindringt, also die Hautbarriere nicht überwinden kann [B. Ponnaiya et al., Far-UVC light prevents MRSA infection of superficial wounds in vivo, PLOS ONE 2018, 13(2): e0192053)]. Eine Ursache hierfür liegt in der Wellenlängenabhängigkeit des Absorptionskoeffizienten der Haut, welcher für Wellenlängen < 250 nm deutlich ansteigt [M. Bounnano et al., 207-nm UV Light - A Promising Tool for Safe Low-Cost Reduction of Surgical Site Infections. I: In Vitro Studies., PLOS ONE 2013, 8(10): e76968]. Daraus folgt, dass in 18 µm Tiefe von der Hautoberfläche UV-Strahlung bei 233 nm etwa 100x stärker abgeschwächt wird als UV-Strahlung bei 254 nm. Die relative Intensität wird dabei um einen Faktor 10⁴ im Vergleich zur Intensität an der Oberfläche abgeschwächt. Die Unterschiede in der Schädigungswirkung von UV-Strahlung beim 222 nm und 254 nm mit unterschiedlicher Dosis wurden bereits bei Basalzellen mit cis-syn cyclobutane pyrinnidine dimer (CPD) an Maushaut nachgewiesen [Saadati S., Study of ultraviolet C light penetration and damage in skin, Columbia Univ/Univ Gothenburg, 2016: https://radfys.gu.se/digitalAssets/1616/1616169_study-of-ultraviolet-c-light-penetration-and-damage-in-skin.pdf].

Aus der WO 2019/077817 A1 ist bekannt, dass eine UV-Antiseptik mit Strahlung aus einem Wellenlängenbereich von 190 nm oder höher bis 230 nm oder geringer dazu geeignet ist, eine antiseptische Wirkung an einem zu dekolonisierenden Zielorganismus zu bewirken, ohne dabei dessen tierische oder menschliche Zellen zu schädigen. Es werden eine entsprechende Vorrichtung und ein Verfahren zur Sterilisation insbesondere bei einer Besiedlung mit Staphylococcus aureus (S.aureus) vorgeschlagen. Die offenbarten Vorrichtungen basieren jedoch auf Gasentladungs- bzw. Excimerlampen und sind daher aufgrund der aufwendigen Lichtleitung bei UV-Strahlung nicht für Anwendungen bei einer klinischen intrakorporalen Antiseptik geeignet. Zudem erzeugen diese erhebliche Mengen an Wärmestrahlung, sodass eine unmittelbare in-vivo Anwendung auf lebender Haut ebenfalls nur bedingt möglich ist.

Davon abgesehen, ist UVC-Strahlung mit Wellenlängen ≤ 230 nm jedoch prinzipiell dazu geeignet, Erreger, insbesondere MRE, an Oberflächen auch innerhalb des Körpers abzutöten, ohne dass Nebenwirkungen zu befürchten sind. Die für eine Abtötung von MRE erforderlichen Strahlendosen liegt hierbei bei < 100 mJ/cm². Bei Patienten mit MRSA-Besiedlung im Nasenraum, Rachen oder in Wunden könnte somit eine von Antiseptika freie Dekolonisierung erreicht werden. Bei einer Kolonisation von MRE, insbesondere von gramnegativen Bakterien und von Enterokokken, in Körperhöhlen wie Nasennebenhöhlen, Uterus, Harnblase oder Darmtrakt ist die Anwendung von Antiseptika nicht möglich. Antibiotika können nur therapeutisch, nicht aber zur Dekolonisation eingesetzt werden. Wünschenswert wäre in solchen Fällen eine lokal Behandlung, welche eine mikrobizide Wirkung vor Ort entfalten kann. Dafür sind jedoch Vorrichtungen und Verfahren erforderlich, die dazu geeignet sind, auch eine intrakorporale in-vivo UV-Antiseptik am menschlichen und tierischen Körper durchzuführen. Insbesondere die aufwendige Wellenleitung im UV-Bereich und die fehlende Möglichkeit zu einer gleichmäßigen und großflächigen lokalen Bestrahlung auch intrakorporaler dreidimensionaler Gewebestrukturen verhindern dabei jedoch bisher eine klinische Anwendung. Eine weitere Schwierigkeit stellt die Wärmeentwicklung der bisher verwendeten Strahlungsquellen dar, welche einen Mindestabstand zwischen der Haut- bzw. Gewebeoberfläche erforderlich machen und dadurch ebenfalls den möglichen Anwendungsbereich einschränkt.

Die EP 3 195 900 A1 betrifft ein Sterilisationsgerät, das durch Bestrahlung mit ultravioletten Strahlen sterilisiert, und insbesondere ein Sterilisationsgerät, das in der Lage ist, den Bestrahlungsbereich der ultravioletten Strahlen sichtbar zu machen. US 2006/0167531 A1 betrifft eine optische Therapievorrichtung zur Bereitstellung von therapeutischem Licht für die Nasenhöhle einer Person, umfassend einen Korpus und mindestens eine UV Lichtquelle in oder an dem Korpus. Die US 2016/0303394 A1 offenbart eine UV-Bestrahlungsvorrichtung zur Erzeugung von UV-Strahlung in einem Wellenlängenbereich von 200-420 nm.

Der Erfindung liegt die Aufgabe zugrunde, die Probleme des Standes der Technik zu überwinden oder zumindest zu verringern und eine Vorrichtung und ein Verfahren insbesondere zur intrakorporalen in-vivo UV-Antiseptik am menschlichen und tierischen Körper zur Verfügung zu stellen. Wesentliches Ziel der Erfindung ist es, eine Vorrichtung zu realisieren, die eine Dekolonisierung verschiedener Oberflächen und Kavitäten insbesondere am Menschen ermöglicht (z.B. Nasenraum incl. Nasennebenhöhlen, Rachen, Wunden), ohne dass das entsprechend behandelte Gewebe (Haut, Schleimhaut) dabei geschädigt wird. Die erfindungsgemäße Aufgabe wird gemäß den unabhängigen Patentansprüchen 1, 6 und 10 gelöst. Bevorzugte Weiterbildungen sind Gegenstand der jeweils rückbezogenen Unteransprüche.

Die Erfindung betrifft eine Vorrichtung zur UV-Antiseptik nach Anspruch 1. Die Vorrichtung umfasst insbesondere einen lichtemittierenden Diodenchip, LED-Chip, dazu ausgebildet, Strahlung im UVC-Spektralbereich zu emittieren, wobei der LED-Chip mit einem Packaging eine lichtemittierende Diode, LED, ausbildet; ein spektrales Filterelement, dazu eingerichtet, die vom LED-Chip emittierte Strahlung im Wesentlichen auf Wellenlängen unterhalb von 235 nm, bevorzugter unterhalb von 230 nm, noch bevorzugter unterhalb von 225 nm, zu beschränken; und ein optisches Element zur gerichteten Abstrahlung der von der LED emittierten Strahlung.

Bei dem LED-Chip kann es sich vorzugsweise um eine Gruppe III-Nitrid UV-LED mit einer auf einem Substrat aus Saphir (Al₂O₃) oder AIN aufgebrachten epitaktischen Schichtstruktur handeln, die im Wesentlichen in einem Wellenlängenbereich von 210 nm bis 240 nm emittiert. Eine UV-LED wird daraus üblicherweise im Flip-Chip-Verfahren aufgebaut, wobei eine Kontaktierung der Kontaktschichten des LED-Chips mittels eines mit Metallelektroden versehenen Submounts erfolgt. Eine Emission der erzeugten Strahlung erfolgt dabei durch das Substrat, d.h. durch die Unterseite des LED-Chips. Der Submount mit dem LED-Chip wird üblicherweise durch Kapselung gegen Umwelteinflüsse geschützt. Diese Kapselung bildet das Packaging, wobei die gekapselten Komponenten dann üblicherweise zusammenfassend als LED bezeichnet werden. Zur gerichteten Abstrahlung der von der LED emittierten Strahlung kann das Packaging ein transparentes optisches Element, z.B. ein Austrittsfenster, eine Austrittslinse oder ein Element zur Strahlformung, umfassen. Da im UV-Bereich eine Auswahl an transparenten Materialien stark eingeschränkt ist, wird für das optische Element üblicherweise hochreines Quarzglas verwendet. Das optische Element kann insbesondere die Kappe der Verkapselung des Packaging bilden. Der Begriff Abstrahlung bezieht sich auf eine unmittelbare Aussendung auf die zu dekolonisierenden Oberflächen, ohne dass danach weitere optische Elemente involviert sind. Der Begriff gerichtet bedeutet insbesondere, dass die Abstrahlung gezielt auf eine zu dekolonisierende Oberfläche erfolgen kann.

Eine erfindungsgemäße Vorrichtung zur UV-Antiseptik kann auch mehrere erfindungsgemäße LEDs umfassen, welche ein gemeinsames LED-Modul ausbilden. Vorzugsweise weist ein solches LED-Modul ein Gehäuse zur Aufnahme der LEDs auf, welche wiederum auf einem gemeinsamen Schaltungsträger (PCB - printed circuit board) angeordnet sein können. Das Gehäuse kann analog zum Packaging ein optisches Element zur gerichteten Abstrahlung der von den LEDs emittierten Strahlung aufweisen. Insbesondere kann es sich hierbei um einen Deckel aus Quarzglas handeln. Das LED-Modul kann mehrere LED-Chips mit oder ohne einem individuellen Packaging umfassen. Im weiteren Sinne kann das Anordnen mehrerer LED-Chips bzw. LEDs innerhalb eines gemeinsamen Gehäuses ebenfalls als Packaging angesehen werden.

Anwendungsmöglichkeiten bieten sich insbesondere im klinischen Bereich bei Patienten mit einer Besiedelung mit MRSA oder andere MRE. Durch eine Dekolonisierung im Nasenraum, Rachen, in Wunden und sonstigen Körperhöhlen mittels UV-LED-Bestrahlung sollte es möglich sein, Infektionen bei Patienten und die weitere Ausbreitung von MRE zu verhindern. Die vorliegende Erfindung beschreibt insbesondere den Einsatz besonders kurzwelliger UVC-Strahlung von LEDs zur direkten Bestrahlung der Haut, Schleimhaut oder Wunden von Patienten, um Mikroorganismen abzutöten, ohne signifikant zu schädigen. Das Wirkprinzip der UV-Antiseptik besteht in der Schädigung der DNA der Mikroorganismen. Die Verträglichkeit des Verfahrens wird über die geringe Wellenlänge des LED-Lichts gewährleistet, infolge derer das Licht nahezu vollständig in der Hornschicht der Haut absorbiert wird und die darunter liegende lebende Epidermis nicht schädigt.

Die auch als DUV-LED-Strahlermodule (DUV - deep ultraviolet) bezeichneten, erfindungsgemäßen Vorrichtungen sind insbesondere zur intrakorporalen in-vivo Antiseptik hervorragend geeignet. Entsprechende LEDs weisen ausreichend hohe Flächenleistungsdichten und ein breites UV-Spektrum auf, welche für eine hohe Wirksamkeit der Bestrahlung bei potentiell unschädlicher Wärmeentwicklung vorteilhaft sind. Die Herausforderung beim Design eines DUV-LED-Strahlermoduls liegt allerdings darin, eine ausreichend hohe Flächenleistungsdichte im Wellenlängenbereich < 235 nm zu erzielen, jedoch gleichzeitig die Emission von längerwelliger UVC- und UVB-Strahlungsanteilen, d.h. von Strahlung mit Wellenlängen > 235 nm, zu vermeiden, da diese in tiefere Hautschichten eindringen und das menschliche Gewebe schädigen können. Um eine effiziente Antiseptik bei kurzen Bestrahlungszeiten zu ermöglichen, sollte die eingestrahlte Leistung möglichst hoch gewählt sein. Bei einer zu hohen Leistungsdichte kann es jedoch, insbesondere aufgrund thermischer Effekte, ebenfalls zu Gewebeschädigungen kommen. Dabei muss beachtet werden, dass die Eigenerwärmung der LEDs bzw. des LED-Moduls, die mit zunehmendem Betriebsstrom (und damit höherer Leistungsdichte), begrenzt werden muss, um eine Gewebeschädigung aufgrund thermischer Effekte zu vermeiden. Es muss daher ein Optimum zwischen der gewählten Grenzwellenlänge (zur Vermeidung von strahlungsbedingten Gewebeschäden) und einer für eine ausreichende Antiseptik erforderlichen Strahlungsdosis gefunden werden.

Untersuchungen mit gefilterten Kr-CI-Excimerlampen (Emission bei 222 nm) zeigen, dass bei dieser Wellenlänge und einer Bestrahlungsdosis von 50 mJ/cm² keine oder kaum Schädigungen im Gewebe auftreten und trotzdem eine Dekolonisierung der Oberfläche beobachtet wird. Auch bei ersten Untersuchungen mit einem frequenzverdoppelten Ar-Laser (Laseremission bei 229 nm) an exzidierter Haut wurden bis zu einer Bestrahlungsdosis von 300 mJ/cm² keine oder kaum Schädigungen im Gewebe gefunden. Während der Einsatz von Excimerlampen durch ihre erhebliche Wärmabstrahlung und damit einhergehender Hautbelastung begrenzt ist, sind UV-LEDs inhärent kalte Strahler bei denen die Verlustwärme effektiv zur hautabgewandten Seite abgeführt und durch eine Begrenzung der elektrischen Leistung kontrolliert werden kann. Die Wärmeabstrahlung einer UV-LED ist insofern unkritisch. Weiterhin ist eine UV-Applikation aufgrund der röhrenförmigen Bauform von Excimerlampen bei flächenmäßig beliebigen bzw. schwer zugänglichen Hautarealen nur eingeschränkt möglich, während sich mittels einer Anordnung aus mehreren UV-LEDs auch flexible Flächenstrahlergeometrien realisieren lassen.

Da die Emissionsspektren von UV-LEDs eine endliche Halbwertsbreite aufweisen (die FWHM ist typischerweise 8-14 nm) und zum Teil zusätzlich noch eine parasitäre Lumineszenz ("Defektlumineszenz") im längerwelligen UV-Bereich auftritt, muss im DUV-LED-Modul zur UV-Antiseptik dafür Sorge getragen werden, dass diese Spektralanteile möglichst niedrige Intensitäten haben bzw. ganz eliminiert werden. Hierfür weist die Vorrichtung ein spektrales Filterelement auf. Eine Möglichkeit zur Umsetzung ist die Integration eines optischen Kurzpassfilters (oder alternativ eines Bandpassfilters mit einer entsprechenden unteren Bandkante bei Verwendung als Kurzpassfilter) in das Packaging bzw. direkt in oder auf den LED-Chip. Ein solcher optischer Kurzpassfilter lässt sich z.B. mittels UV-transparenter dielektrischer Schichtstapel realisieren. Bei einer Gruppe III-Nitrid-basierten UV-LED könnte ein solcher Kurzpassfilter beispielsweise aus 10 AlOₓ/SiO₂-Spiegelpaaren aufgebaut sein. Ein solcher optischer DBR (DBR - distributed Bragg reflector) kann direkt während der Epitaxie im LED-Chip integriert werden. Der DBR kann jedoch auch auf dem LED-Chip aufgebracht werden, etwa indem man die Spiegelpaare auf die Rückseite eines polierten Saphirsubstrates des LED-Chips abscheidet. Eine andere Möglichkeit ist die Integration der Spiegelpaare in das UV-LED Packaging, z.B. als Teil einer Quarzglasverkappung des Packagings. Beim Design eines zur spektralen Verteilung der Emission eines LED-Chips passenden spektralen Filterelements können auch die Seitenbandenreflektivitäten angepasst (z.B. chirped DBR mirror) oder die Breite des Stoppbandes (z.B. andere Dielektrika wie HfO₂) beeinflusst werden.

Die Reflektivität eines AlOₓ/SiO₂-DBR kann beispielsweise derart eingestellt werden, dass diese bei 233 nm minimal ist und für längere UV-Wellenlängen sehr schnell ansteigt (d.h. diese Wellenlängen werden herausgefiltert). Aufgrund einer praktisch limitierten Anzahl an möglichen Filterschichten wird es sich bei dem Kurzpass im Allgemeinen um eine Bandsperre mit einer entsprechenden Bandflanke bei der gewünschten Sperrwellenlänge handeln. Mit 10 AlOₓ/SiO₂-Spiegelpaaren lassen sich jedoch bereits Sperrbreiten von etwa 60 nm erreichen, sodass ein solches Filterelement im Wesentlichen transparent für Wellenlängen unterhalb von 233 nm und reflektierend (d.h. sperrend) für Wellenlängen zwischen 233 nm und etwa 293 nm ausgebildet werden kann. Dies ist zur Vermeidung von Hautschädigungen ausreichend, da das typische Emissionsspektrum einer DUV-LED mit einer Maximalintensität bei 233 nm im Wesentlichen auf Wellenlängen zwischen 220 nm und 260 nm beschränkt ist.

Die notwendige Strahlungsdosis für die log₁₀ Dekolonisierung ist ebenfalls wellenlängenabhängig (und auch unterschiedlich für verschiedene Mikroorganismen). Für die UV-Antiseptik von E.coli liegt das Maximum des Wirkungsspektrums bei 265 nm bis 270 nm und nimmt zu kürzeren Wellenlängen hin ab. Um bei einem E.coli-Bakterium eine log₁₀ Reduktion zu erzielen, wird bei einer Wellenlänge von 265 nm eine Bestrahlungsdosis von 7 mJ/cm² benötigt, während bei 230 nm bereits eine Bestrahlungsdosis von 20 mJ/cm² erforderlich ist. Das Bakterium S.aureus hat im Vergleich zu E.coli eine leicht geringere Deaktivierungskonstante *k* (E.coli: *k* = 0,10575 m²/J, S.aureus: *k* = 0,07132 m²/J, beide für eine Oberflächenbestrahlung mit einer Bestrahlungswellenlänge von 254 nm), sodass für eine log₁₀ Reduktion bei 230 nm mit einer etwas höheren Bestrahlungsdosis von 30 mJ/cm² gerechnet werden muss [W. Kowalski, "Ultraviolet Germicidal Irradiation Handbook", Springer 2009].

Die Maximalleistung von UV-LEDs mit einer Maximalintensität bei einer Wellenlänge von 233 nm liegt aktuell bei etwa 1,5 mW, wobei diese über einen Zeitraum von 100 Stunden typischerweise auf 30% bis 50% der Ausgangsleistung abnimmt und anschließend nur noch geringfügig abfällt, d.h. die langzeitstabile Leistung liegt aktuell maximal bei etwa 0,5 mW. Auch mit dieser relativ geringen Leistung ist es jedoch möglich, ein im klinischen Umfeld einsetzbares DUV-LED-Modul zu realisieren. Bei einer Einstrahlung der Leistung auf eine Fläche von einem Quadratzentimeter beträgt die Flächenleistungsdichte 0,5 mW/cm². Um eine Bestrahlungsdosis von 30 mJ/cm² zu erreichen, wird somit eine Bestrahlungszeit von maximal 60 s, d.h. etwa eine Minute, benötigt. Dies ist bereits ein praktikabler Zeitraum für eine klinische UV-Antiseptik. Es ist jedoch davon auszugehen, dass mit der Verbesserung der LED-Technologie in Zukunft noch deutlich kürzere Bestrahlungszeiten möglich werden. Eine Erhöhung der abgegebenen Leistung um mindestens eine Größenordnung, z.B. mittels Wachstum von DUV-LEDs auf bulk-AIN, erscheint hierbei realistisch. Die notwendige Bestrahlungszeit würde sich dann auf unter 6 s reduzieren und eine klinische Anwendung wäre somit sogar operationsbegleitend möglich. Bisher ist nämlich kein Verfahren bekannt, mit welchem postoperative Wundinfektionen im OP-Umfeld (surgical side infection, SSI) effektiv reduziert werden können. Insbesondere bei lang andauernden Operationen ist eine antiseptische Zwischenbehandlung zumeist nicht möglich. Eine operationsbegleitende Strahlungsantiseptik könnte effektiv zur Verhinderung einer Besiedelung der operierten Person mit Krankheitserregern beitragen. Sofern eine Dekolonisation durch MRSA oder andere MRE präoperativ nicht möglich bzw. nicht gelungen ist, kommt einer die OP begleitenden Antiseptik im OP-Umfeld besondere Bedeutung zu.

Eine weitere Möglichkeit die Lichtleistung von DUV-LEDs zu erhöhen, ist die LED gepulst zu betreiben. Durch die Ansteuerung mit sehr kurzen elektrischen Pulsen (einige 100 ns bis zu 1 µs) lassen sich Erwärmungseffekte innerhalb des LED-Chips vermeiden. Die LEDs können dadurch bei höheren Strömen oder Stromdichten betrieben werden, wodurch sich eine hohe Pulsenergie erreichen lässt. Dies kann zur Erhöhung der maximalen Durchschnittsleistung bzw. für eine Steigerung der Deaktivierungseffizienz genutzt werden. Durch eine gepulste Bestrahlung kann die notwendige Bestrahlungszeit auch mit aktuellen DUV-LEDs gegenüber einem kontinuierlichen Dauerbetrieb reduziert werden.

Ein Problem bei der Integration von optischen Filtern und UV-LEDs ist die Tatsache, dass die Emission der LEDs über alle Raumwinkel erfolgt. Dabei wird bei einer im Flip-Chip-Verfahren aufgebauten UV-LED das UV-Licht typischerweise in den oberen Halbraum durch das UV-transparente Substrat (z.B. Saphir) hindurch in alle Raumwinkel emittiert. Da die Lichtstrahlen bei einer ebenen Schichtstruktur des spektralen Filterelements je nach Einfallswinkel unterschiedliche effektive Schichtdicken durchlaufen müssen, folgt daraus, dass die Grenzwellenlänge (auch als Cut-Off-Wellenlängen bezeichnet) der dielektrischen Kurzpassfilter (oder entsprechend genutzter Bandpassfilter) mit dem Einfallswinkel der UV-Strahlen variiert. Für eine optimale Filterung sollte daher diese winkelabhängige Verschiebung der Grenzwellenlänge der dielektrischen Kurzpassfilter bzw. Bandpassfilter vermieden werden.

Vorzugsweise kann die Form des spektralen Filterelements an die Abstrahleigenschaften der LEDs angepasst werden. Insbesondere kann dies dadurch erreicht werden, dass die einzelnen Spiegelschichten eines oben beschriebenen spektralen Filterelements (DBR) auf eine halbkugelförmige Kappe (bzw. einen Deckel) aus z.B. UV-transparentem Quarzglas oder Silicon aufgebracht werden. In diesem Fall ist die von der Strahlung durchlaufene effektive Schichtdicke für alle Raumrichtungen gleich. Bei einer Zeilenanordnung von mehreren LEDs kann stattdessen auch eine halbzylinderförmige Kappe (Deckel) verwendet werden. Eine Winkelunabhängigkeit kann dann zumindest in Achsen senkrecht zur Zeilenausrichtung realisiert werden.

Weitere Ansätze für winkelunabhängige Filter sind durch Yi-Jun Jen et al. (Design and Fabrication of a Narrow Bandpass Filter with Low Dependence on Angle of Incidence, Coatings, 8(7):231 (2018)) und L. Lin et al. (Angle-robust resonances in cross-shaped aperture arrays, Applied Physics Letters 97, 061109 (2010)) bekannt.

Bei Yi-Jun Jen et al. wird ein nahezu winkelunabhängiger Bandpassfilter über die Anregung von lokalisierten Oberflächenplasmonen (localized surface plasmons) in kreuzförmigen Öffnungen in 140 nm dicken Silberschichten auf Glas realisiert. Die kreuzförmigen Öffnungen haben eine Länge von ca. 240 nm und sind mit einer Periode von 360 nm in 2 Dimensionen angeordnet. Allerdings ist das Design für einen Filter im infraroten Bereich ausgelegt (Bandpass bei einer Wellenlänge von ca. 1000 nm). Vorzugsweise wird ein solches Design auf UV-Wellenlängen übertragen und als spektrales Filterelement im Rahmen dieser Erfindung eingesetzt. Die Dimension der Kreuzstrukturen und die Periode müssen dazu jedoch deutlich kleiner ausgelegt werden, vorzugsweise um einen Faktor 3 bis 5. Zudem werden anstatt Silber andere Metalle mit einer höheren Plasmafrequenz, z.B. Aluminium, bevorzugt zur Herstellung verwendet. Ein entsprechendes spektrales Filterelement kann dann z.B. auf UV-transparentes Quarzglas oder ein anderes UV-transparentes dielektrisches Material aufgebracht werden.

L. Lin et al. zeigen ebenfalls einen nahezu winkelunabhängigen Bandpassfilter. Das Fabry-Perot-Filterkonzept (FP-Filterkonzept) basiert hier auf der Kombination von Metallschichten und Dielektrika als Spiegelschichten. Es wurde ein aus 5 Schichten bestehender FP-Filter mit der Sequenz Luft/Ag(13 nm)/Silizium(90 nm)/Ag(10 nm)/Silizium(97 nm)/Ag(20 nm) auf Glas abgeschieden. Diese Schichtfolge ermöglicht einen nahezu winkelunabhängigen Bandpassfilter im infraroten Spektralbereich bei einer Wellenlänge von 900 nm. Vorzugsweise wird auch dieses Design auf UV-Wellenlängen übertragen und als spektrales Filterelement im Rahmen dieser Erfindung eingesetzt. Die Schichtdicken müssen hierbei entsprechend angepasst sein, d.h. vorzugsweise um einen Faktor 3 bis 5 verkleinert. Zur Herstellung werden vorzugsweise anstatt Silber andere Metall mit einer höheren Plasmafrequenz, z.B. Aluminium, verwendet. UV transparente Dielektrika (Al₂O₃ oder HfO₂, CaF₂) können dann auf z.B. UV-transparentes Quarzglas oder ein anderes UV-transparentes dielektrisches Material aufgebracht werden.

Vorzugsweise kann für eine Überwachung der auf die zu dekolonisierende Oberfläche eingestrahlten Strahlungsleistung mindestens eine Monitordiode (Photodiode) als Detektor in die lichtemittierende Diode oder die Vorrichtung integriert sein. Dies ermöglicht eine kontinuierliche Überwachung der emittierten bzw. abgestrahlten Leistung und kann insbesondere zur Feststellung eines Defektes der Vorrichtung oder einer fehlerhaften Anwendung genutzt werden.

Vorzugsweise werden die räumlichen Abstrahleigenschaften der LED über die Breite, Zusammensetzung und Verspannung der Bandstruktur des LED-Chips, die äußere Geometrie des LED-Chips oder durch in das Packaging integrierte Reflektoren und Blenden bestimmt. Entsprechende Freiheiten in der Bauform der UV-LEDs und darauf basierender Strahler erlauben es nämlich, die räumliche Abstrahlcharakteristik definiert einzustellen. Dies ist speziell für die Anwendung bei der in-vivo-Bestrahlung von großem Nutzen. Um beispielsweise die schädigende Wirkung der UVC-Strahlung auf die Haut zu minimieren, kann zum einen die Wellenlänge möglichst klein gehalten werden, zum anderen kann die Einstrahlung unter mehr streifendem als senkrechtem Einfall auf die Haut erfolgen. Während die Reduktion der Wellenlänge deutlich unterhalb von 230 nm aufgrund der damit einhergehenden Abnahme der Strahlungsleistung problematisch ist, kann der Einfallswinkel durch strahlungsgeometrische Freiheiten beim Design der LED bzw. eines darauf basierenden Strahlers beeinflusst werden.

Weiterhin ist bei der Bestrahlung von Flächen die räumliche Uniformität der Strahlungsleistung wichtig, um die Wirksamkeit des Verfahrens über die bestrahlte Fläche sicherzustellen. Beim Einstrahlen in Körperöffnungen sollte hingegen die Abstrahlung möglichst uniform über den gesamten Raumwinkel erfolgen, um die innere Oberfläche der Körperöffnung vollständig ausleuchten zu können. Um Erreger auf einer topologisch komplexen Körperoberfläche zu eradizieren, sind flache Abstrahlwinkel erforderlich, um abschattende Strukturen zu unterstrahlen. Es ist daher bevorzugt, dass das erfindungsgemäße optische Element an die jeweilige Bestrahlungssituation angepasst ist bzw. angepasst werden kann. Beispielsweise ist es vorteilhaft, wenn die Abstrahlung innerhalb von Körperhöhlen derart erfolgt, dass die Körperhöhle weitgehend formschlüssig durch das optische Element ausgefüllt wird und eine Bestrahlung über die gesamte in der Körperhöhle befindliche Elementstruktur erfolgt. Durch das möglichst formschlüssige Ausfüllen kann ein einheitlicher Abstand zwischen der zu bestrahlenden Oberfläche und dem optische Element eingestellt werden.

Die räumliche Abstrahlung von UV-LEDs und darauf basierender Strahler kann auf vielfältige Art und Weise individuell angepasst werden. Die räumliche Abstrahlung des LED-Chips hängt unter anderem auch von der Bandstruktur der aktiven Zone ab. Durch deren Breite, Zusammensetzung und Verspannung kann die Bandstruktur (und damit auch die Polarisation des UV-Lichts) beeinflusst werden. Eines dieser Merkmale gilt dabei immer dann als bestimmend, wenn es für die räumliche Abstrahlung (z.B. Öffnungswinkel der Strahlung) das begrenzende Merkmal darstellt. Die äußere Geometrie des UV-LED-Chips, insbesondere die Größe der Seitenflächen des Chips, bestimmt die Winkelabstrahlcharakteristik und kann ebenfalls entsprechend eingestellt werden. Im Packaging einer UV-LED können Reflektoren, Blenden, Linsen und/oder ein geformter Verguss verschiedener Geometrie integriert werden, welche die Abstrahlung ebenfalls beeinflussen können. Bei Flächenstrahlern erlauben eine flexible Anordnung der UV-LEDs sowie die Verwendung von Reflektoren im Strahler eine Homogenisierung der Intensität über die bestrahlte Fläche.

Vorzugsweise ist das spektrale Filterelement als intrinsischer Bragg-Reflektor innerhalb des LED-Chips als auf den LED-Chip aufgebrachter Bragg-Reflektor oder als in das Packaging integrierter Bragg-Reflektor ausgebildet. Für einen intrinsischen, innerhalb des LED-Chips angeordneten Bragg-Reflektor können mehrere AlₓGa₁₋ₓN/Al_{y}Ga_{1-y}N-Schichtpaare (x ≠ y) in die Epitaxieschichtstruktur der UV-LED integriert werden. Bei einem auf dem LED-Chip aufgebrachten Bragg-Reflektor kann es sich um einen auf dem Substrat aufgebrachten Schichtstapel handeln. Ein in das Packaging integrierter Bragg-Reflektor kann auf die transparente Kappe (z.B. Quarzkappe) einer Verkapselung aufgebracht sein. Alternativ kann das spektrale Filterelement als externes optisches Bauteil innerhalb des Packaging angeordnet sein. Bei einer Anordnung mehrerer UV-LEDs in einem gemeinsamen Gehäuse kann das spektrale Filterelement analog sowohl auf einem zugehörigen transparenten Deckel (z.B. Quarzdeckel) als auch als externes optisches Bauteil innerhalb des Gehäuses angeordnet sein.

Vorzugsweise umfasst das optische Element eine Linse, eine Lightpipe oder eine optische Faser. Wie bereits oben angegeben, kann es sich bei dem optischen Element um ein einfaches Austrittsfenster, z.B. in Form der Quarzkappe eines Packagings, handeln. Insbesondere für die flächige Beleuchtung einer Oberfläche kann der Austrittsbereich, d.h. der von der emittierten Strahlung transmittierte Bereich, auch eine Linse zur Strahlformung bzw. -kollimation umfassen. Diese kann beispielsweise in eine Quarzkappe oder einen Quarzdeckel integriert oder auch extern innerhalb des Packaging bzw. in einem Gehäuse angeordnet sein. Eine Lightpipe kann dazu genutzt werden, die emittierte Strahlung in unmittelbar von außen erreichbare Körperöffnungen, wie den Rachenraum oder die Nasenhöhlen, einzubringen. Mit einer optischen Faser als optisches Element kann die emittierte Strahlung auch gezielt an schwer erreichbaren Körperstellen bzw. -öffnungen, z.B. während sog. minimalinvasiver Operationen, appliziert werden. Bevorzugtes Material für die optischen Elemente ist dabei Quarzglas.

Die Kopplung einer UV-LED an Lightpipes ist insbesondere für Bestrahlungen im Nasen- bzw. Rachenraum vorgesehen. Um eine möglichst effiziente Kopplung des von der UV-LED emittierten Lichtes zu ermöglichen, sollte die Lightpipe möglichst nahe bei der UV-LED positioniert werden. Möglich ist auch eine Stoßkopplung zwischen Lightpipe und UV-LED, die bevorzugt mit UV-transparenten Klebematerialien (z.B. Schott DUV 200) unterstützt wird. Es können dabei eine oder mehrere LEDs zur Beleuchtung benutzt werden, eventuell auch Arrays aus UV-LEDs, die in einem gemeinsamen Packaging aufgebaut wurden. Im einfachsten Fall kann eine Lightpipe als Hohlleiter ausgeführt sein, dessen innere Oberfläche (z.B. durch eine Beschichtung mit Aluminium) im UV-Bereich reflektierend ist. Die bevorzugte Verwendung flexibler, d.h. biegsamer, Materialien erlaubt es, die Lightpipe einfach an die Körperöffnung heranzuführen bzw. innerhalb verwinkelter Körperhohlräume gut an die bevorzugt zu bestrahlenden Areale heranzukommen.

Bei gefüllten Lightpipes sollte das Füllmaterial UV-transparent sein, bevorzugt im Wellenlängenbereich zwischen 210 nm und 240 nm. Als Material für Lightpipes und andere UV-transparente Elemente eignen sich u.a. Quarzglas (z.B. Suprasil, Ultrasil, Infrasil), CaF₂, MgF₂ oder Saphir (Al₂O₃). Die Geometrie der Lightpipes kann ergonomisch den Umgebungen im Nasen- und Rachenraum angepasst sein. Der strahlungsführende Bereich außerhalb der Nase bzw. Mundes kann mit einer UV-reflektierenden Schicht bedeckt sein (z.B. Aluminium oder Teflon), welche verhindert, das UV-Strahlung in die Umgebung entweichen kann. In diesem Bereich können auch eine oder mehrere Monitordioden (Photodioden) platziert werden, um die UV-Strahlungsleistung und dessen Verteilung bestimmen bzw. überwachen zu können. Um die UV-Strahlung gezielt am Ende der Lightpipe austreten zu lassen, können lokal Streuzentren in die Lightpipe integriert werden. Im Falle eines Lichtwellenleiters aus Quarz oder ähnlichem können solche Lichtstreuer u.a. über eine lokale Aufrauung der Materialoberfläche realisiert werden.

Ähnlich kann das Design einer fasergekoppelten UV-LED aufgebaut sein, welches insbesondere für die Applikation an schwer zugänglichen Stellen innerhalb des menschlichen Körpers (z.B. Ohren, Harnröhre, Blase, Uterus, ...) geeignet ist. Die optische Faser kann teilweise mit einem (optional UV-reflektierenden) nicht UV-transparenten Mantel umgeben sein, wodurch an diesen Stellen keine UV-Strahlung in die Umgebung entweichen kann. Weiterhin kann das Licht aus mehreren fasergekoppelten UV-LEDs in eine gemeinsame optische Faser zusammengekoppelt werden, um die verfügbare Leistung zu erhöhen. Zudem ist auch hierbei die Integration einer Monitordiode (Photodiode) als Detektor möglich, um die UV-Strahlungsleistung kontinuierlich während des Betriebs bestimmen zu können.

Vorzugsweise ist das optische Element auswechselbar. Dies hat den Vorteil, dass eine erfindungsgemäße Vorrichtung auch an unterschiedliche Aufgaben angepasst werden kann, ohne dass eine weitere Strahlungsquelle erforderlich wäre. Somit kann die Vorrichtung bei gleichbleibender Strahlungsquelle sowohl für großflächige als auch für punktuelle Bestrahlungen genutzt werden. Insbesondere ist dadurch auch ein Wechsel zwischen einer äußeren Anwendung auf der Haut einer Person und einer intrakorporalen Anwendung innerhalb des Körpers einer Person möglich. Ein weiterer Vorteil ist, dass das optische Element nach einer Behandlung mit konventionellen Methoden gereinigt und dekolonisiert werden kann. Insbesondere bei Lightpipes und optischen Fasern kann durch einen schnellen Wechsel bzw. eine unkomplizierte Aufbereitung eine hohe Verfügbarkeit bei der Benutzung der Vorrichtung sichergestellt werden. Eine Auswechselbarkeit des optischen Elements kann über eine entsprechende Kopplung erreicht werden. Auch können nur einmal zu verwendende optische Elemente verwendet werden.

Ein weiterer Aspekt der vorliegenden Erfindung betrifft eine medizinische Auflage zur UV-Antiseptik, umfassend eine Vielzahl von zu einem Array angeordneten erfindungsgemäßen Vorrichtungen, wobei die Vorrichtungen auf einem flexiblen oder einem starren Trägerelement angeordnet sind. Eine solche Auflage kann beispielsweise als eine Art Verband unmittelbar auf ein äußeres Hautareal aufgebracht werden und zur flächigen UV-Antiseptik des bestrahlten Areals genutzt werden. Während sich die Auflage bei einem starren Trägerelement nicht der Körperform anpasst, kann mittels eines flexiblen Trägers (z.B. aus einem Kunststoff- oder Gewebefilm) auch eine entsprechende Anpassung der Form der Auflage erfolgen. Bei einer entsprechenden Größe der Auflage können somit beispielsweise ein Bein oder ein Arm von der Auflage nahezu formschlüssig umschlossen werden. Ein starres Trägerelement hat jedoch den Vorteil, dass es eine einfache Anwendung der erfindungsgemäßen Auflage erlaubt.

Vorzugsweise umfasst das Trägerelement ein flexibles oder starres Abstandselement, welches dazu ausgebildet ist, im auf einer zu dekolonisierenden Oberfläche aufgelegten Zustand einen Abstand zwischen den zu einem Array angeordneten Vorrichtungen und der Oberfläche einzustellen, wobei das Abstandselement eine Bestrahlung der Oberfläche mit der von den Vorrichtungen emittierten Strahlung ermöglicht. Durch ein Abstandselement kann ein fest vorgegebener Abstand zwischen der bestrahlten Oberfläche und den LEDs in der Auflage eingestellt werden. Dadurch ist der Abstand nicht vom Anwender abhängig und auf die Oberfläche kann eine genau definierte Strahlungsdosis eingestrahlt werden. Des Weiteren kann eine Kontamination der LEDs vermieden werden, da eine Hautberührung nur über das Abstandselement erfolgt. Insbesondere lässt sich dadurch auch eine Verunreinigung bzw. Kontamination des optischen Elements vermeiden.

Vorzugsweise ist das Abstandselement auswechselbar mit dem Trägerelement verbunden. Dies hat den Vorteil, dass das Abstandselement mit gängigen Verfahren dekolonisiert bzw. sterilisiert werden kann. Dadurch können eventuell mit Infektionserregern kontaminierte Abschnitte des Abstandselements, d.h. insbesondere die mit der Haut einer behandelten Person in Berührung gekommenen Auflagestellen, vor einer Weiterbenutzung der Auflage gereinigt werden. Es können auch mehrere Abstandselemente im Wechsel oder zur einmaligen Verwendung genutzt werden. Dadurch wird eine hohe Verfügbarkeit bei der Benutzung der Auflage gewährleistet.

Vorzugsweise ist für eine Überwachung der auf die zu dekolonisierende Oberfläche eingestrahlten Strahlungsleistung mindestens eine Monitordiode als Detektor in die Auflage integriert. Über die Monitordiode (Photodiode) kann die von der Auflage abgestrahlte Leistung überwacht werden. Mittels einer Vielzahl von über die Auflage verteilten Monitordioden kann zudem geprüft werden, ob eine einheitliche Flächenhelligkeit vorhanden ist.

Vorzugsweise kann die UV-Strahlungsverteilung dadurch homogenisiert werden, dass die Hülle der Auflage innen mit einer UV-reflektierenden Schicht versehen ist (z.B. Aluminium, Teflon). Nach außen hin sollte die Hülle UV-undurchlässig sein, um die Umgebung vor austretender UV-Strahlung zu schützen.

Ein weiterer, nicht in den Ansprüchen enthaltener Aspekt der Erfindung betrifft ein Verfahren zur in-vivo Antiseptik menschlicher Haut, wobei eine Bestrahlung der Oberfläche der Haut mit einer von einer UV-LED im UVC-Bereich emittierten Strahlung erfolgt, wobei das Spektrum der Strahlung mittels eines spektralen Filterelements im Wesentlichen auf Wellenlängen unterhalb von 235 nm, bevorzugter unterhalb von 230 nm, noch bevorzugter unterhalb von 225 nm, beschränkt ist. Vorzugsweise liegt das Intensitätsmaximum der emittierten Strahlung des LED-Chips bei einer Wellenlänge unterhalb von 235 nm, bevorzugter unterhalb von 230 nm, noch bevorzugter unterhalb von 225 nm.

Die für die Vorrichtung genannten zusätzlichen Merkmale und bevorzugten Weiterbildungen gelten für das erfindungsgemäße Verfahren entsprechend. Ein solches Verfahren kann auch zur Behandlung chronischer Wunden genutzt werden. Chronische Wunden sind ein bevorzugter Rückzugsort von MRSA und bisher mit Strahlungstherapien nicht behandelbar. Dies liegt insbesondere daran, dass bei chronischen Wunden durch eine begleitende Entzündung des jeweiligen Trägerorgans (z.B. Haut) das Gewebe sehr empfindlich auf äußere Einflüsse reagiert und beispielsweise ein zusätzlich durch die Behandlung bedingter Wärmeeintrag (z.B. Verlustwärme einer Excimerlampe) eine unzumutbare Belastung für eine zu behandelnde Person darstellen würde.

In einem weiteren Aspekt der Erfindung wird ein Verfahren zur UV-Antiseptik offenbart, welches das Bestrahlen einer zu dekolonisierenden Oberfläche mit einer erfindungsgemäßen Vorrichtung (bzw. einer erfindungsgemäßen Auflage) umfasst. Die für die Vorrichtung bzw. die Auflage genannten zusätzlichen Merkmale und bevorzugten Weiterbildungen gelten für das erfindungsgemäße Verfahren entsprechend.

Weitere bevorzugte Ausgestaltungen der Erfindung ergeben sich aus den übrigen, in den Unteransprüchen genannten Merkmalen.

Die verschiedenen in dieser Anmeldung genannten Ausführungsformen der Erfindung sind, sofern im Einzelfall nicht anders ausgeführt, mit Vorteil miteinander kombinierbar.

Die Erfindung wird nachfolgend in Ausführungsbeispielen anhand der zugehörigen Zeichnungen erläutert. Es zeigen:
- Figur 1: eine schematische Darstellung einer typischen Schichtstruktur eines Gruppe III-Nitrid-basierten UV-LED-Chips auf einem Submount,
- Figur 2: ein Reflexionsspektrum eines AlOₓ/SiO₂-DBR mit 10 Spiegelschichten (linkes Diagramm) sowie das Emissionsspektrum einer typischen UV-LED mit einem solchen spektralen Filterelement (rechtes Diagramm),
- Figur 3: schematische Darstellungen unterschiedlicher Ausführungsformen zur Integration eines spektralen Filterelements,
- Figur 4: schematische Darstellungen unterschiedlicher Ausführungsformen von erfindungsgemäßen Vorrichtungen,
- Figur 5: eine schematische Darstellung einer ersten Ausführungsform einer erfindungsgemäßen Auflage,
- Figur 6: eine schematische Darstellung einer zweiten Ausführungsform einer erfindungsgemäßen Auflage, und
- Figur 7: eine schematische Darstellung einer Ausführungsform zur Integration eines vom Abstrahlwinkel unabhängigen spektralen Filterelements.

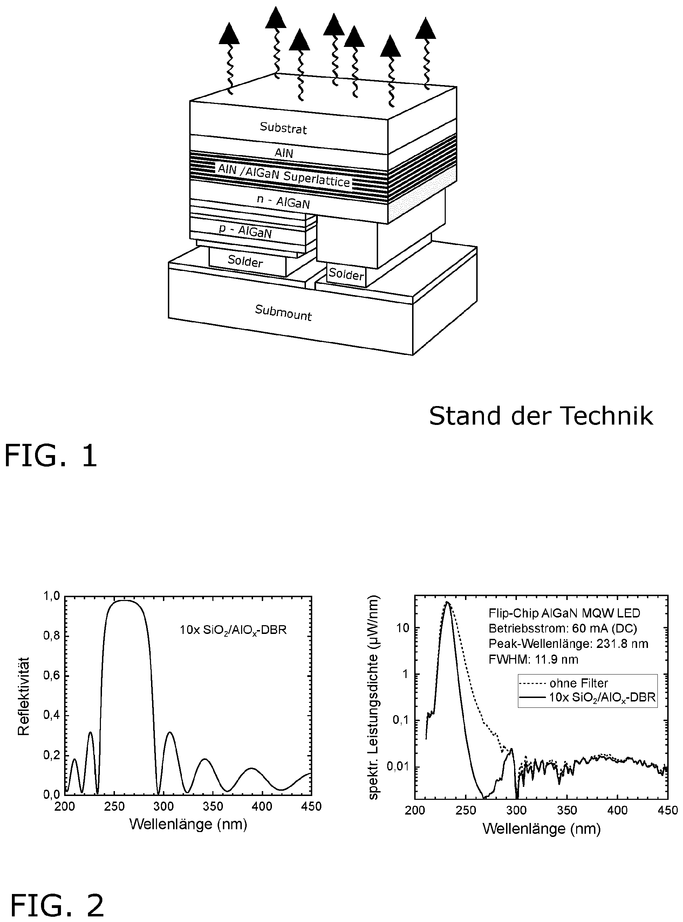

Figur 1 zeigt eine schematische Darstellung einer typischen Schichtstruktur eines Gruppe III-Nitrid-basierten UV-LED-Chips auf einem Submount. Als Substrat zur Herstellung der Schichtstruktur wird zumeist entweder Saphir (Al₂O₃) oder AIN verwendet. Beide Substratmaterialen sind im Prinzip UV-transparent, sodass die in den üblichen AlₓGa₁₋ₓN-Quantenfilmen entstehende UV-Strahlung effizient durch das Substrat ausgekoppelt werden kann. Durch Wahl des Aluminiumgehaltes in den AlₓGa₁₋ₓN-Quantenfilmen und den typischerweise umgebenden Al_{y}Ga_{1-y}N-Quantenbarrieren sowie der Breite der AlₓGa₁₋ₓN-Quantenfilmen lässt sich die Emissionswellenlänge der UV-LED einstellen. Für Emissionswellenlängen im Bereich von 210 nm bis 240 nm liegen die Aluminiumgehalte in den Quantenfilmen zwischen x = 0,99 und x = 0,6 und die Zusammensetzung der Barrieren zwischen y = 1 und y = 0,65. Die Breite der Quantenfilme variiert üblicherweise zwischen 0,5 nm bis 20 nm, wobei typischerweise eine Quantenfilmdicke von etwa 1 nm verwendet wird. Die Zahl der Quantenfilme liegt zwischen N = 1 und N = 20, typischerweise bei N = 3. Um eine Lichtextraktion durch das Substrat zu ermöglichen, muss auch eine eventuell vorhandene Al_{z}Ga_{1-z}N-Stromspreizschicht UV-transparent sein. Der Aluminiumgehalt in der Al_{z}Ga_{1-z}N-Stromspreizschicht sollte daher so gewählt sein, dass diese Schicht eine Bandlücke aufweist, die größer als die Photonenenergie der UV-Strahlung ist, d.h. die Zusammensetzung liegt bevorzugt im Bereich von z = 1 bis z = 0,65.

Die Breite des Emissionsspektrums einer UV-LED lässt sich ebenfalls durch das Heterostrukturdesign sowie eine Variationen in der Materialzusammensetzung und bei den einzelnen Schichtdicken beeinflussen. Die Einstellbarkeit der Emissionswellenlänge der UV-LED ermöglicht es, das Emissionsspektrum der UV-LED optimal für die jeweilige Anwendung anzupassen. Daher kann erfindungsgemäß beispielsweise ein möglichst optimaler Kompromiss zwischen der emittierten Leistung der UV-LED, der erzielten antiseptische Wirkung und der Vermeidung von Schädigung des bestrahlten Gewebes eingestellt werden. Mit abnehmender Wellenlänge sinken typischerweise auch die Leistung und Effizienz der UV-LEDs, d.h. die antiseptische Wirkung wird geringer bzw. die Bestrahlungszeit muss erhöht werden, um die gleiche Dosis zu erreichen. Allerdings dringt die UV-Strahlung bei kürzeren Wellenlängen nicht so tief in die Gewebeoberfläche ein, d.h. die Schädigung ist geringer. Die Leistung und Effizienz der UV-LEDs steigen mit zunehmender Wellenlänge deutlich an, d.h. die antiseptische Wirkung wird stärker. Allerdings dringt die UV-Strahlung bei längeren UV-Wellenlängen tiefer in die Gewebeoberflächen ein, d.h. die Schädigung ist größer. Zudem umfasst das typische Emissionsspektrum einer UV-LED auch einen längerwelligen Anteil, der möglicherweise eine schädigende Wirkung auf das bestrahlte Gewebe haben könnte, insbesondere Wellenlängenanteile im Bereich > 240 nm, die in tiefere Hautschichten eindringen können.

Figur 2 zeigt ein Reflexionsspektrum eines AlOₓ/SiO₂-DBR mit 10 Spiegelschichten (linkes Diagramm) sowie das Emissionsspektrum einer typischen UV-LED mit einem solchen spektralen Filterelement (rechtes Diagramm).

Das links dargestellte Reflexionsspektrum wurde mittels der Transfermatrixmethode für den senkrechten Einfall berechnet. Es handelt sich um ein spektrales Filterelement, bestehend aus 10 AlOₓ/SiO₂-Spiegelpaaren, welche einen DBR (DBR - Distributed Bragg Reflector) ausbilden. Für entsprechende Brechungsindizes und Dicken bei den einheitlichen Schichten wurden bei AlOₓ *n* = 1,85 und *d* = 35,16 nm bzw. für SiO₂ *n* = 1,46 und *d* = 44,52 nm angenommen. Die Reflektivität des AlOₓ/SiO₂-DBR ist minimal bei 233 nm (R = 0,7%) und steigt für längere UV-Wellenlängen sehr schnell an (d.h. diese Wellenlängen werden herausgefiltert). Bei einer Wellenlänge von 240 nm beträgt die Reflektivität bereits R = 82 %, bei 250 nm beträgt R = 97 %.

Die Steilheit des Stoppbandes (DBR) lässt sich durch die Zahl der Spiegelpaare einstellen, d.h. die Verwendung von mehr Spiegelpaaren führt zu einem steileren Stoppband. Zudem lässt sich die Breite des Stoppbandes (DBR) durch die Wahl der einzelnen Dielektrika kontrollieren. Ein höherer Unterschied im Brechungsindex der Dielektrika führt dabei zu einer Verbreiterung des Stoppbandes (DBR).

Das rechts dargestellte Diagramm zeigt das Emissionsspektrum einer typischen UV-LED in logarithmischer Darstellung mit und ohne den vorstehend beschriebenen spektralen Filter. Für die UV-LED ohne Filter (gestrichelte Kurve) liegt die Peakwellenlänge in diesem Beispiel bei 231,8 nm und die Halbwertsbreite bei 11,9 nm. Durch die Integration eines voranstehend beschriebenen Filterelements lässt sich der längerwellige UV-Anteil des Emissionsspektrums deutlich reduzieren, ohne dass sich die spektrale Leistung im kurzwelligen UV-Bereich merklich verringert. Die Peakwellenlänge der UV-LED mit dem Filter (durchgehende Kurve) verschiebt leicht auf 232,2 nm, die Halbwertsbreite nimmt deutlich auf 7,7 nm ab. Insbesondere der längerwellige UV-Anteil mit Wellenlängen > 240 nm wird stark reduziert.

Die Breite des UV-LED Emissionsspektrum lässt sich auch durch das Heterostrukturdesign sowie eine Variationen in der Materialzusammensetzung und bei den einzelnen Schichtdicken beeinflussen.

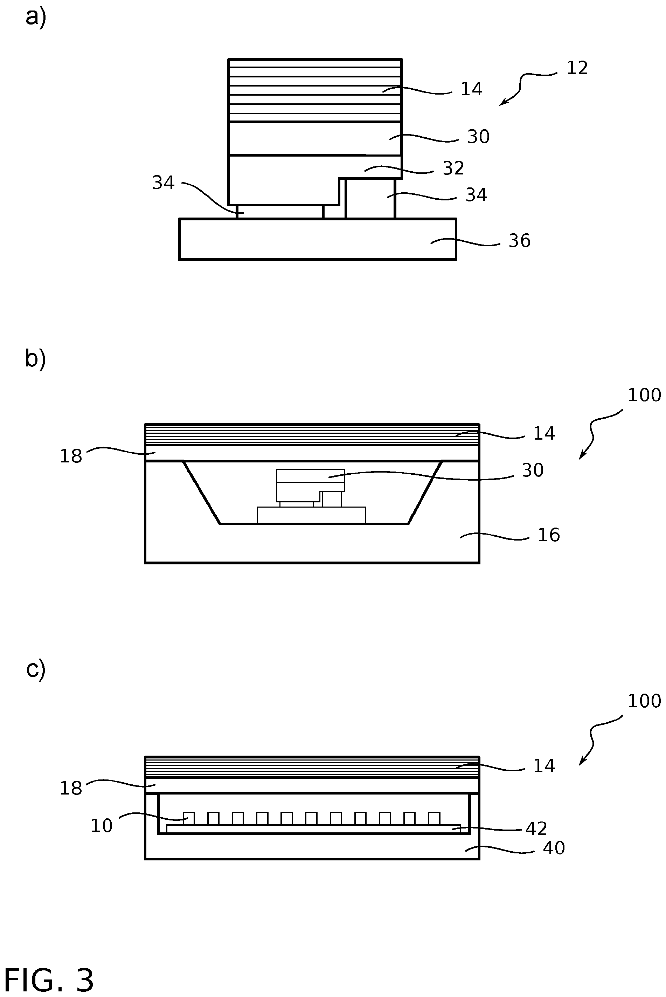

Figur 3 zeigt schematische Darstellungen unterschiedlicher Ausführungsformen zur Integration eines spektralen Filterelements 14.

In Abbildung a) ist der typische Aufbau eines UV-LED-Chips 12 auf einem Submount 36 dargestellt. Die aktive Schicht 32 des LED-Chips 12 ist dabei über Kontakte 34 elektrisch leitend mit dem Submount 36 verbunden. Die Verbindung erfolgt typischerweise über ein Flip-Chip-Verfahren, d.h. das Substrat 30 des LED-Chips 12, auf dem die entsprechende Halbleiterschichtstruktur aufgewachsen wurde, befindet sich oberhalb der aktiven Schicht 32 und wird zur Strahlungsauskopplung von seiner Unterseite durchstrahlt. In der gezeigten Ausführungsform zur Integration eines spektralen Filterelements 14 wurde dieses auf die freie Oberseite des Substrats 30 aufgebracht. Das spektrale Filterelement 14 und die aktive Schicht 32 liegen sich somit an unterschiedlichen Seiten des Substrats 30 gegenüber. Alternativ kann das spektrale Filterelement 14 auch auf der Seite der aktiven Schicht 32, d.h. innerhalb oder angrenzend an die aktive Schicht 32, angeordnet sein.

Die Abbildung b) zeigt den vorstehend beschriebenen LED-Chip 12 auf einem Submount 36 (ohne aufgebrachtes spektrales Filterelement) mit einem Packaging 16. Das Packaging 16 schützt den LED-Chip 12 vor Verschmutzung und Beschädigung. Die Oberseite des Packaging kann eine für UV-Strahlung transparente Kappe aufweisen (z.B. Austrittsfenster aus Quarzglas). Auf diese Kappe kann das spektrale Filterelement 14 aufgebracht werden. Dies entspricht einem Aufbringen auf das Substrat 30 entsprechend Abbildung a). In der gezeigten Abbildung soll die Kappe auch zur gerichteten Abstrahlung der emittierten Strahlung dienen und stellt somit gleichzeitig ein erfindungsgemäßes optisches Element 18 dar. Die gezeigte erfindungsgemäße Vorrichtung 100 könnte somit unmittelbar für eine lokale UV-Antiseptik genutzt werden.

In Abbildung c) sind mehrere UV-LEDs 10 (oder LED-Chips 12) zu einem Modul verbunden. Unter einer LED 10 wird dabei ein LED-Chip 12 mit einem Packaging 16 verstanden, ohne dass ein spektrales Filterelement 14 oder ein erfindungsgemäßes optisches Element 18 vorhanden sein müssen (die Abdeckung der einzelnen Packagings stellt hier lediglich ein inneres Bauteil dar und dient nicht der Abstrahlung). Die einzelnen LEDs 10 können auf einem gemeinsamen Schaltungsträger 42 (z.B. PCB - printed circuit board, Leiterplatte) in einem Gehäuse 40 angeordnet sein. Wie das Packaging in Abbildung b) kann das Gehäuse 40 einen für UV-Strahlung transparenten Deckel aufweisen (z.B. Austrittsfenster aus Quarzglas). Falls die Abstrahlung unmittelbar durch den Deckel erfolgt, handelt es sich hierbei um ein erfindungsgemäßes optisches Element 18. Auf den Deckel kann das spektrale Filterelement 14 aufgebracht werden. Das beschriebene Modul stellt somit lediglich ein Multi-LED-Packaging dar, das zugrundeliegende Prinzip entspricht dem in Abbildung b). Daher stellt diese Ausführungsform lediglich eine erweiterte Form des Packaging mit mehreren LEDs 10 bzw. LED-Chips 12 dar.

Hinsichtlich des spektralen Filterelements ist zu beachten, dass die FWHM-Halbwertsbreite der Emissionsspektren von UV-LEDs (FWHM - Full-Width-Half-Maximum) im Allgemeinen zwischen 5 nm und 25 nm, typischerweise bei 10-12 nm, liegt. Die integrierte Emissionsleistung zwischen 210 nm und 235 nm beträgt knapp 300 µW und die Gesamtleistung über alle Wellenlängen etwa 475 µW. Um eine Schädigung des Gewebes durch tief eindringendes UV-Licht zu vermeiden, sollten die längerwelligen Anteile (> 235 nm) möglichst aus dem Spektrum eliminiert werden. Dies kann wie vorstehend beschrieben insbesondere durch die Integration eines spektralen Filterelements als optischer Kurzpassfilter (Kurzpassfilter bezüglich der Wellenlänge) in das Modul bzw. direkt in den UV-LED-Chip (z.B. auf die freie Substratseite) oder das Packaging geschehen. Das spektrale Filterelement kann allerdings auch als zusätzliches Element in ein LED-Modul integriert werden. Ein solcher optischer Kurzpassfilter kann z.B. als DBR (distributed Bragg reflector) mittels eines Schichtstapels aus UV-transparenten Schichten mit unterschiedlichen Brechungsindizes realisiert werden. Dafür können insbesondere mehrere AlₓGa₁₋ₓN/Al_{y}Ga_{1-y}N-Schichtpaare (x ≠ y) mit in die Epitaxieschichtstruktur der UV-LED integriert werden. Weiterhin können Schichtpaare aus Dielektrika mit unterschiedlichem Brechungsindex auf die Rückseite des Substrats oder in das Packaging (z.B. auf einer Quarzkappe) integriert werden. Andere Dielektrika, die sich für die Herstellung von DBR-Filtern eignen, sind z.B. HfO₂, Ta₂O₅, CaF₂, MgF₂, Ga₂O₃ oder AIN. Die spektralen Eigenschaften des Kurzpassfilters können insbesondere durch die Wahl der geeigneten Materialien sowie die Dicke und Zahl der DBR-Spiegelschichten an die jeweilige Anwendung optimiert werden.

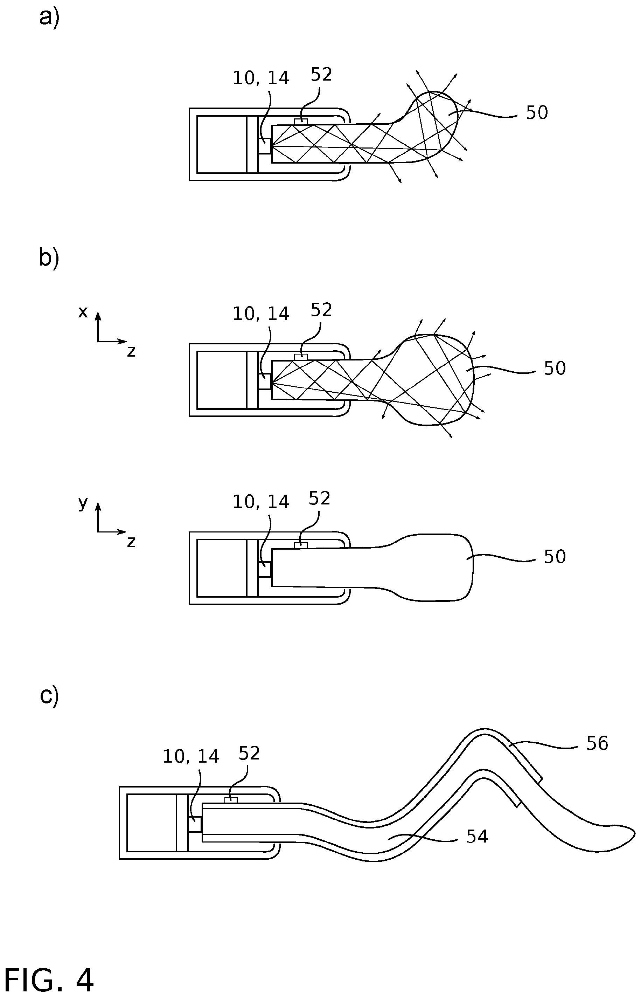

Figur 4 zeigt schematische Darstellungen unterschiedlicher Ausführungsformen von erfindungsgemäßen Vorrichtungen 100. Alle Ausführungsformen können optional eine oder mehrere Monitordioden 52 zur Überwachung der Strahlungsleistung aufweisen. Die grundlegende Idee dieser Ausführungsformen ist, durch gezielte Ausbildung des erfindungsgemäßen optischen Elements 18 die Abstrahlung möglichst optimal an die zu dekolonisierenden Areale anzupassen. Hierzu werden insbesondere körperangepasste Lightpipes 50 sowie optische Fasern 54 verwendet. Die Darstellung ist nicht einschränkend in Bezug auf die Verwendung eines einzelnen Emitters auszulegen. Beispielsweise kann anstelle der gezeigten Kombination einer LED 10 und eines spektralen Filterelements 14 auch jede der in Figur 3 gezeigten Ausführungsformen zur Integration eines spektralen Filterelements 14 verwendet werden.

In Abbildung a) ist eine exemplarische Darstellung einer Vorrichtung 100 zur UV-Antiseptik für Anwendungen im Nasenraum gezeigt. Das erfindungsgemäße optisches Element 18 umfasst eine Lightpipe 50, welche dazu ausgebildet ist, die von einer UV-LED 10 erzeugte und mit einem spektralen Filterelement 14 gefilterte Strahlung zielgerichtet in den Nasenraum einer zu behandelnden Person zu leiten. Bei der Lightpipe 50 kann es sich insbesondere um einen massiven oder als Hohlleiter ausgebildeten Quarzglasstab handeln. Die Lightpipe 50 kann ein UV-transparentes Füllmaterial umfassen. Die Form der Lightpipe 50 ist dabei vorzugsweise in Größe und Form der zu behandelnden Körperöffnung angepasst, hier dem Nasenraum bzw. der Nasenhöhle. Für den Nasenraum entspricht dies einem geraden Abschnitt zum Einführen in ein Nasenloch und einem geneigt dazu angeordneten kugelförmigen Abschnitt zum Ausfüllen der Nasenhöhle. Um ausschließlich eine lokale Bestrahlung des Nasenraums zu ermöglichen, kann der Austrittsbereich der Strahlung eingeschränkt sein. Zudem ist es vorteilhaft, wenn die Lightpipe 50 gegen andere Lightpipes 50 mit unterschiedlicher Größe und Form auswechselbar ist. Dadurch kann eine optimale Anpassung der Bestrahlung an den jeweiligen Nutzer erfolgen.

In Abbildung b) ist eine exemplarische Darstellung einer Vorrichtung 100 zur UV-Antiseptik für Anwendungen im Rachenraum gezeigt. Die Darstellung entspricht weitgehend der unter a) gezeigten, die Bezugszeichen gelten entsprechend. Im Unterschied zur Darstellung unter a) variiert jedoch die Form der Lightpipe 50. Insbesondere weist sie entsprechend dem Aufbau eines typischen menschlichen Rachenraums eine unterschiedliche Gestaltung in der Aufsicht (gerader Abschnitt mit sich gerade anschließendem ballonförmigen Abschnitt) und in der Seitenansicht (gerader Abschnitt mit sich gerade anschließendem spatelförmigen Abschnitt) auf. Auch hierbei ist es vorteilhaft, wenn die Größe und Form der Lightpipe 50 durch einen Austausch angepasst werden kann.

In Abbildung c) ist eine exemplarische Darstellung einer Vorrichtung 100 zur UV-Antiseptik für Anwendungen in schwer zugänglichen Stellen oder dünnen Kanälen gezeigt. Die Darstellung entspricht wiederum weitgehend der unter a) gezeigten, die Bezugszeichen gelten entsprechend. Im Unterschied zur Darstellung unter a) handelt es bei dem optischen Element 18 jedoch nicht um eine Lightpipe 50, sondern um eine UV-transparente optische Faser 54 (z.B. aus Quarzglas). Solche Fasern haben den Vorteil, dass diese flexibel sind und gebogen werden können. Die Fasern können somit auch in ansonsten nur schwer zugängliche Stellen, beispielsweise in üblicherweise nicht nach außen offenen Körperhohlräumen, eingeführt werden. Die Spitze der Faser kann dabei eine zur Bestrahlung bevorzugte Form aufweisen, z.B. plan, kugelförmig oder spitz ausgebildet sein. Um zu verhindern, dass die UV-Strahlung bereits bei der Zuleitung aus der Faser austritt, kann ein für UV-Strahlung intransparenter Mantel 56 um einen der LED 10 zugewandten Teil der Faser vorhanden sein.

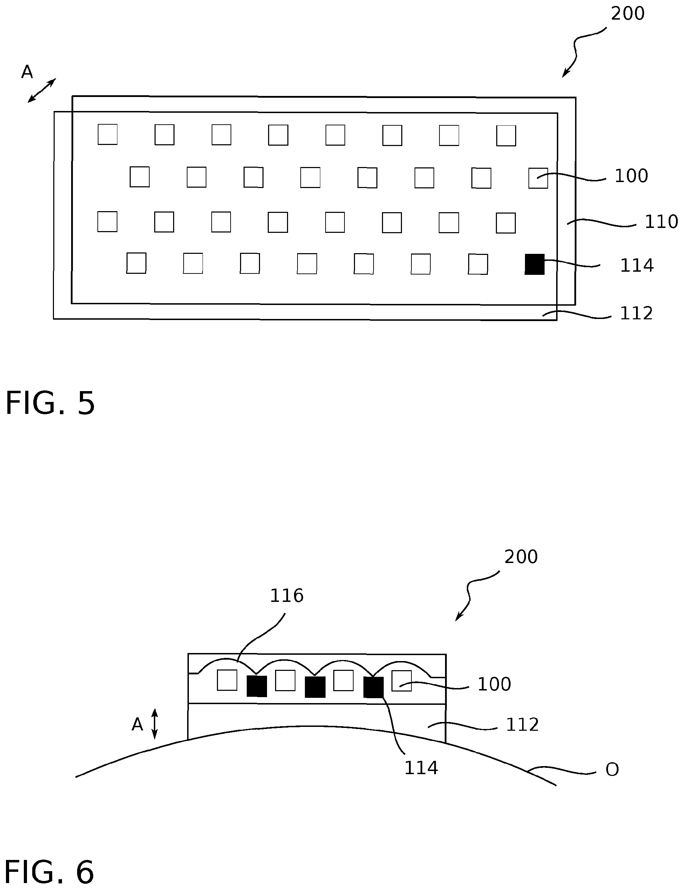

Figur 5 zeigt eine schematische Darstellung einer ersten Ausführungsform einer erfindungsgemäßen Auflage 200. Diese umfasst eine Vielzahl von zu einem Array angeordneten erfindungsgemäßen Vorrichtungen 100, wobei die Vorrichtungen 100 auf einem flexiblen oder einem starren Trägerelement 110 angeordnet sind. Insbesondere kann es sich hierbei um ein flexibles Trägerelement 110 (z.B. ein Stoffgewebe) handeln. Die elektrische Kontaktierung der einzelnen Vorrichtungen 100 kann bei Geweben mittels eingewebter flexibler Zuleitungen erfolgen. Das Trägerelement 110 kann ein flexibles oder starres Abstandselement 112 umfassen, welches dazu ausgebildet ist, im auf einer zu dekolonisierenden Oberfläche O aufgelegten Zustand einen Abstand A zwischen den zu einem Array angeordneten Vorrichtungen 100 und der Oberfläche O einzustellen, wobei das Abstandselement 112 eine Bestrahlung der Oberfläche O mit der von den Vorrichtungen 100 emittierten Strahlung ermöglicht. Das Abstandselement 112 kann dabei insbesondere aus hygienischen Gründen auswechselbar mit dem Trägerelement 110 verbunden sein. Weiterhin kann für eine Überwachung der auf die zu dekolonisierende Oberfläche O eingestrahlten Strahlungsleistung mindestens eine Monitordiode 114 als Detektor in die Auflage 200 integriert sein.

Figur 6 zeigt eine schematische Darstellung einer zweiten Ausführungsform einer erfindungsgemäßen Auflage 200. Die Darstellung entspricht weitgehend der in Figur 5 gezeigten, die Bezugszeichen gelten entsprechend. Im Unterschied zur Darstellung in Figur 5 ist hier jedoch die Seitenansicht einer erfindungsgemäßen Auflage 200 mit einer Vielzahl von zusätzlichen Reflektoren 116 gezeigt. Insbesondere kann es sich hierbei um eine Auflage mit starrem Trägerelement 110 (z.B. ein (Kunststoff-)Gestell) handeln. Das Abstandselement 112 ist hier exemplarisch mit einer gekrümmten Auflagefläche dargestellt. Dies ermöglicht beispielsweise eine unmittelbare Auflage auf eine entsprechend gekrümmte Oberfläche. Sowohl die Reflektoren 116 als auch die freie Ausgestaltung der Auflagefläche des Abstandselements 112 sind allerdings auch bei flexiblen Trägerelementen 110 realisierbar.

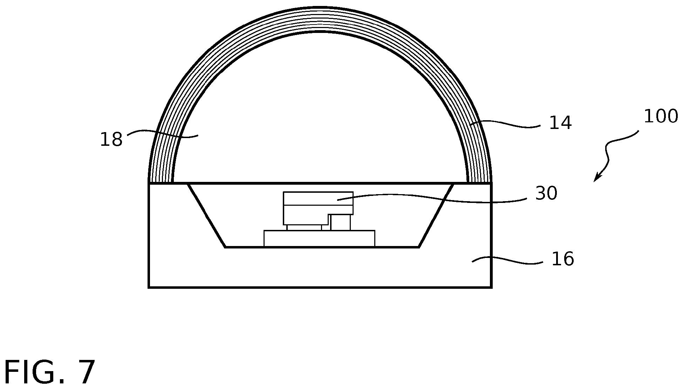

Figur 7 zeigt eine schematische Darstellung einer Ausführungsform zur Integration eines vom Abstrahlwinkel unabhängigen spektralen Filterelements. Die Darstellung entspricht weitgehend der in Figur 3 b) gezeigten, die Bezugszeichen gelten entsprechend. Im Unterschied zur Darstellung in Figur 3 b) variiert jedoch die Form der als optisches Element 18 verwendeten transparenten Kappe. Diese ist hier insbesondere kuppelförmig ausgebildet und befindet sich oberhalb des Packagings 16. In der gezeigten Abbildung stellt die Kappe gleichzeitig ein erfindungsgemäßes optisches Element 18 dar. Bei der Kappe kann es sich jedoch alternativ auch um ein internes optisches Element (Fenster bzw. Linse) einer erfindungsgemäßen Vorrichtung handeln.

Die Kappe kann dabei vorzugsweise als Halbkugel aus z.B. UV-transparentem Quarzglas oder Silicon ausgebildet sein. Die einzelnen Schichten des spektralen Filterelements 14 können dabei unmittelbar auf die gekrümmte Oberfläche der Kappe aufgebracht werden. Dadurch wird erreicht, dass die UV-Lichtstrahlen aus dem LED-Chip 12 größtenteils senkrecht auf die DBR-Spiegelschichten auftreffen und sich die Grenzwellenlänge des spektralen Filterelements 14 nicht mit dem Abstrahlwinkel verändert. Ein weiterer Vorteil ist, dass durch eine solche Konfiguration die Lichtextraktion aus der UV-LED verbessert wird und sich somit die Effizienz und die Ausgangsleistung steigern lassen. Eine gleichmäßige Beschichtung der halbkugelförmigen Kappe lässt sich insbesondere durch eine geeignete Halterung in einer Beschichtungsanlage bzw. durch Drehung der Kappe während des Beschichtungsprozesses erreichen.

Werden mehrere UV-LEDs 10 (oder LED-Chips 12) entsprechend Figur 3 c) zu einem Modul verbunden, kann das spektrale Filterelement 14 auch auf eine halbzylinderförmig ausgebildete Kappe (bzw. einen Deckel) aus z.B. UV-transparenten Quarzglas oder Silicon aufgebracht werden. Die in Figur 7 gezeigte Darstellung würde sich dabei in die Darstellungsebene hinein erstrecken, wobei die Schichtfolge des spektralen Filterelements 14 in der Tiefe unverändert ist. Der Nachteil einer solchen zylinderförmigen Anordnung ist, dass ein Teil der emittierten UV-Strahlung weiterhin unter einem Winkel auf das spektrale Filterelement auftrifft. Als Vorteil ergibt sich jedoch, dass auch LED-Zeilenarrays mit an die Abstrahleigenschaften angepassten spektralen Filterelementen 14 bestückt werden können und dass eine homogene Beschichtung des Halbzylinders mit Dielektrika relativ einfach zu implementieren ist (z.B. durch eine Rotation des Halbzylinders bei der Beschichtung).

Nachfolgend werden ergänzend die Ergebnisse von Untersuchungen zur Strahlungs- und Dosisabhängigkeit der UV-Antiseptik an exzidierten Hautproben angegeben.

Die Untersuchungen erfolgten an einem frequenzverdoppelten Argon-Ionen-Laser (LEXEL-Laser, 95-SHG) bei einer Wellenlänge von 229 nm. Hierfür stand exzidierte Humanhaut nach einer Narbenentfernung zur Verfügung. Die Probe wurde am Vortrag des Experimentes frisch entnommen. Die Haut wurde mit 0,2 mW/cm² für 5 Minuten und für 30 Minuten bestrahlt. Die Leistung wurde mit einem Leistungsmessgerät bestimmt. Die entsprechenden Strahlungsdosen waren 60 mJ/cm² und 300 mJ/cm². Zusätzlich wurden die 300 mJ/cm² durch 1 mW/cm² bei einer Bestrahlungsdauer von 5 min aufgebracht. Als positive Kontrolle wurde UVB-Strahlung mit den gleichen Dosen eingesetzt. Eine unbestrahlte Probe diente als Negativkontrolle.

Von den Proben wurde jeweils eine 4 mm Stanze in eine Einbettkassette überführt. Eine Fixierung des Gewebes fand durch eine 4%-ige Formaldehydlösung statt. Die Proben wurden am gleichen Tag zur Analyse ins Labor gebracht. Aus den fixierten Gewebeproben wurden Paraffinschnitte (Dicke 1-2 µm) angefertigt und diese mit Hämatoxylin und Eosin angefärbt, um eine Unterscheidung zwischen den unterschiedlichen Gewebestrukturen zu ermöglichen. Mit entsprechenden Antikörpern wurden dann typische DNA-Schäden durch UV-Strahlung, sogenannte Cyclobutan-Pyrimidin-Dimere (CPD) und 6-4 Photoprodukte (6-4PP) nachgewiesen. Zirka 70 % bis 80 % der UV-induzierten DNA-Schäden sind CPDs, die restlichen sind 6-4PPs und deren Isomerformen, die Dewar Photoprodukte. Beide Veränderungen der DNA durch UVC- und UVB-Bestrahlung sollen insbesondere in der Basalmembran verhindert werden. Eine deutliche Färbung der Positivkontrolle gegenüber den eigentlichen Proben zeigte deutlich, dass Strahlung bei 229 nm im Gegensatz zu UVB-Strahlung keine bis wenig CPDs in den Zellen erzeugt. In Vergrößerung sind bei 300 mJ/cm² "CPD-Zellen" in der oberen Epidermis zu finden. Die zweite Bestrahlung bei 300 mJ/cm² weist keinerlei solche Zellen auf. Bei einer UVB-Bestrahlung treten die CPD-Schäden hingegen sehr eindeutig und intensiv bis in die oberen Dermisschichten auf. Weiterhin konnten mit einer UV-Bestrahlung bei 229 nm unabhängig von der Strahlungsdosis keinerlei 6-4PP-Schäden gefunden werden. Im Gegensatz dazu sind diese bei einer UVB-Bestrahlung stark ausgeprägt.

Durch diese Untersuchungen konnte somit erfolgreich gezeigt werden, dass bei einer UV-Bestrahlung bei 229 nm nur wenig bis keine DNA-Schäden an exzidierten Hautproben auftreten und selbst hohe Dosen von 300 mJ/cm² die lebende Epidermis nur sehr oberflächlich schädigen.

Weiterhin wurden Untersuchungen an Schweineohren zur Keimreduktion durchgeführt. Auf markierten Arealen eines Schweineohrs wurden folgende antiseptische Einwirkungen verglichen:
- Ethanol basiertes Hautantiseptikum (AHD 2000), Einwirkungszeit 1 min,
- UVC 60 mJ/cm², (0,2 mW/cm², 5 min),
- UVC 300 mJ/cm² (0,2 mW/cm², 30 min),
- UVC 300 mJ/cm² (1 mW/cm², 2,5 min) und
- unbehandelte Kontrolle.

In der unbehandelten Kontrolle waren auf Grund der geringen Kolonisationsdichte nur in der Probenmenge von 1 ml 15 Kolonie bildende Einheiten nachweisbar. Durch alle antiseptischen Behandlungsmodi wurde die Hautflora komplett eliminiert, ohne dass eine Schädigung des Gewebes erkennbar war. Damit wurde nachgewiesen, dass der vorgesehene Strahlenbereich mikrobiozid wirksam ist.

### Bezugszeichenliste

- 10: LED
- 12: LED-Chip
- 14: spektrales Filterelement
- 16: Packaging
- 18: optisches Element
- 30: Substrat
- 32: aktive Schicht
- 34: Kontakte
- 36: Submount
- 40: Gehäuse
- 42: Schaltungsträger
- 50: Lightpipe
- 52: Monitordiode
- 54: Glasfaser
- 56: Mantel

- 100: Vorrichtung
- 110: Trägerelement
- 112: Abstandselement
- 114: Monitordiode
- 116: Reflektor

- 200: Auflage

- A: Abstand
- O: Oberfläche

## Patentansprüche

1. Vorrichtung (100) zur UV-Antiseptik, insbesondere auf Haut, Schleimhäuten, Wunden, in Körperhöhlen und auf operativ freigelegten Geweben und Organen, umfassend:
einen lichtemittierenden Diodenchip, LED-Chip (12), dazu ausgebildet, Strahlung im UVC-Spektralbereich zu emittieren, wobei der LED-Chip (12) mit einem Packaging (16) eine lichtemittierende Diode, LED (10), ausbildet, wobei der LED-Chip (12) auf einem Substrat aus bulk-AIN oder Saphir aufgewachsen ist;
ein spektrales Filterelement (14), dazu eingerichtet, die vom LED-Chip (12) emittierte Strahlung durch eine weitgehend winkelunabhängige spektrale Filterung im Wesentlichen auf Wellenlängen unterhalb von 235 nm zu beschränken,
wobei das spektrale Filterelement (14) als AlOₓ/SiO₂-DBR oder als DBR-Filter mit HfO₂, Ta₂O₅, CaF₂, MgF₂, Ga₂O₃ oder AlN als Dielektrikum in das Packaging (16) der LED (10) integriert ist, wobei die Form des spektralen Filterelements (14) an die Abstrahleigenschaften der LED (10) angepasst ist; oder
wobei das spektrale Filterelement (14) als winkelunabhängiger Bandpassfilter über die Anregung von Oberflächenplasmonen oder ein Fabry-Pérot-Filterkonzept realisiert wird; und
ein optisches Element (18) zur gerichteten Abstrahlung der von der LED (10) emittierten Strahlung, wobei eine räumliche Uniformität der Strahlungsleistung durch Anpassung der Abstrahlung an eine mittels der Vorrichtung (100) zu bestrahlende Fläche erreicht wird.

2. Vorrichtung (100) nach Anspruch 1, wobei der LED-Chip (12) für eine gepulste Emission mit elektrischen Pulsen einer Dauer von einigen 100 ns bis zu 1 µs angesteuert wird.

3. Vorrichtung (100) nach Anspruch 1 oder 2, wobei die räumlichen Abstrahleigenschaften der LED (10) über die Breite, Zusammensetzung und Verspannung der Bandstruktur des LED-Chips (12), die äußere Geometrie des LED-Chips (12) oder durch in das Packaging (16) integrierte Reflektoren, Blenden, Linsen und/oder geformten Verguss bestimmt sind.

4. Vorrichtung (100) nach einem der vorhergehenden Ansprüche, wobei die Vorrichtung (100) als Flächenstrahler ausgebildet ist und durch eine flexible Anordnung der LED (10) sowie die Verwendung von Reflektoren im Flächenstrahler eine Homogenisierung der Intensität über die zu bestrahlende Fläche erfolgt.

5. Vorrichtung (100) nach einem der vorhergehenden Ansprüche, wobei das optische Element (18) eine Linse, eine Lightpipe (50) oder eine optische Faser (54) umfasst.

6. Vorrichtung (100) nach einem der vorhergehenden Ansprüche, wobei das optische Element (18) auswechselbar ist.

7. Medizinische Auflage (200) zur UV-Antiseptik, umfassend eine Vielzahl von zu einem Array angeordneten Vorrichtungen (100) nach einem der vorhergehenden Ansprüche, wobei die Vorrichtungen (100) auf einem flexiblen oder einem starren Trägerelement (110) angeordnet sind.

8. Medizinische Auflage (200) nach Anspruch 7, wobei das Trägerelement (110) ein flexibles oder starres Abstandselement (112) umfasst, dazu ausgebildet, im auf einer zu dekolonisierenden Oberfläche (O) aufgelegten Zustand einen Abstand (A) zwischen den zu einem Array angeordneten Vorrichtungen (100) und der Oberfläche (O) einzustellen, wobei das Abstandselement (112) eine Bestrahlung der Oberfläche (O) mit der von den Vorrichtungen (100) emittierten Strahlung ermöglicht.

9. Medizinische Auflage (200) nach Anspruch 8, wobei das Abstandselement (112) auswechselbar mit dem Trägerelement (110) verbunden ist.

10. Medizinische Auflage (200) nach einem der Ansprüche 7 bis 9, wobei für eine Überwachung der auf die zu dekolonisierende Oberfläche (O) eingestrahlten Strahlungsleistung mindestens eine Monitordiode (114) als Detektor in die Auflage (200) integriert ist.

## Claims

1. A device (100) for UV antisepsis, in particular on skin, mucous membranes, wounds, in body cavities and on surgically exposed tissues and organs, comprising:
a light-emitting diode chip (LED chip (12)) configured to emit radiation in the UVC spectral range, wherein the LED chip (12) forms a light-emitting diode (LED (10)) with a package (16), wherein the LED chip (12) has been grown on a substrate of bulk AlN or sapphire;
a spectral filter element (14) set up substantially to limit the radiation emitted by the LED chip (12) to wavelengths below 235 nm by means of a largely angle-independent spectral filtering,
wherein the spectral filter element (14) is integrated as an AlOₐ/SiO₂-DBR or as a DBR filter with HfO₂, Ta₂O₅, CaF₂, MgF₂, Ga₂O₃ or AlN as dielectric into the package (16) of the LED (10), wherein the shape of the spectral filter element (14) is adapted to the emission characteristics of the LED (10); or
wherein the spectral filter element (14) is obtained as an angle-independent bandpass filter via excitation of surface plasmons or a Fabry-Pérot filter concept; and
an optical element (18) for directional emission of the radiation emitted by the LED (10), wherein spatial uniformity of the radiant power is achieved by adapting emission to an area to be irradiated by means of the device (100).

2. The device (100) according to Claim 1, wherein the LED chip (12) is driven for pulsed emission with electrical pulses of a duration of a few 100 ns up to 1 µs.

3. The device (100) according to Claim 1 or 2, wherein the spatial emission characteristics of the LED (10) are determined by the width, composition and distortion of the band structure of the LED chip (12), the external geometry of the LED chip (12) or by reflectors, apertures, lenses and/or shaped potting integrated into the package (16).

4. The device (100) according to any one of the preceding claims, wherein the device (100) is configured as a large-area radiator and intensity is homogenized over the area to be irradiated by a flexible arrangement of the LEDs (10) and the use of reflectors in the large-area radiator.

5. The device (100) according to any one of the preceding claims, wherein the optical element (18) comprises a lens, a light pipe (50) or an optical fiber (54).

6. The device (100) according to any one of the preceding claims, wherein the optical element (18) is replaceable.

7. A medical pad (200) for UV antisepsis, comprising a plurality of devices (100) according to any one of the preceding claims arranged to form an array, wherein the devices (100) are arranged on a flexible or a rigid support element (110).

8. The medical pad (200) according to Claim 7, wherein the support element (110) comprises a flexible or rigid spacer element (112) configured to establish a distance (A) between the devices (100) arranged to form an array and the surface (O) when placed on a surface (O) to be decolonized, wherein the spacer element (112) allows irradiation of the surface (O) with the radiation emitted by the devices (100).

9. The medical pad (200) according to Claim 8, wherein the spacer element (112) is replaceably connected to the support element (110).

10. The medical pad (200) according to any one of Claims 7 to 9, wherein at least one monitor diode (114) is integrated into the pad (200) as a detector for monitoring the radiant power irradiated onto the surface (O) to be decolonized.

## Revendications

1. Dispositif (100) destiné à une antisepsie par rayonnement ultraviolet - UV, en particulier sur la peau, des muqueuses, des plaies, dans des cavités corporelles et sur des tissus et organes dégagés chirurgicalement, comprenant :
une puce de diode électroluminescente, puce de DEL *- LED-Chip* (12), configurée pour émettre un rayonnement dans la bande spectrale UV-C, la puce de DEL (12) configurant avec un encapsulage (16) une diode électroluminescente, DEL - *LED* (10), la puce de DEL (12) ayant été mise en croissance sur un substrat en nitrure d'aluminium - *bulk-AlN,* ou saphir ;
un élément filtrant spectral (14), mis en place pour limiter le rayonnement émis par la puce de DEL (12) par un filtrage spectral de manière largement indépendante des angles et sensiblement à des longueurs d'ondes inférieures à 235 nm,
dans lequel l'élément filtrant spectral (14) est intégré sous la forme d'un filtre AlOₓ/SiO₂-DBR ou d'un filtre DBR avec HfO₂, Ta₂O₅, CaF₂, MgF₂, Ga₂O₃ ou AlN comme milieu diélectrique dans l'encapsulage (16) de la DEL (10), la forme de l'élément filtrant spectral (14) étant adaptée aux propriétés d'irradiation de la DEL (10) ; ou
dans lequel l'élément filtrant spectral (14) est réalisé sous la forme d'un filtre passe-bande indépendant des angles sur l'excitation de plasmons de surface ou un concept de filtre de Fabry Pérot ; et
un élément optique (18) destiné à l'irradiation directionnelle du rayonnement émis par la DEL (10), dans lequel une uniformité spatiale de la puissance de rayonnement est atteinte en adaptant l'irradiation sur une surface à irradier à l'aide du dispositif (100).

2. Dispositif (100) selon la revendication 1, dans lequel la puce de DEL (12) est actionnée pour une émission pulsée avec des impulsions électriques d'une durée de quelques 100 ns jusqu'à 1 µs.

3. Dispositif (100) selon la revendication 1 ou 2, dans lequel les propriétés d'irradiation spatiale de la DEL (10) sont définies par la largeur, la composition et la tension de la structure en ruban de la puce de DEL (12), la géométrie externe de la puce de DEL (12) ou par des réflecteurs, des ouvertures, des lentilles et/ou un composé d'enrobage profilé intégré dans l'encapsulage (16).

4. Dispositif (100) selon l'une des revendications précédentes, dans lequel le dispositif (100) est configuré sous la forme d'un émetteur de rayonnement de surface et une disposition flexible de la DEL (10) ainsi que l'utilisation de réflecteurs dans l'émetteur de rayonnement de surface permettent une homogénéisation de l'intensité sur la surface à irradier.

5. Dispositif (100) selon l'une des revendications précédentes, dans lequel l'élément optique (18) comprend une lentille, un guide de lumière (50) ou une fibre optique (54).

6. Dispositif (100) selon l'une des revendications précédentes, dans lequel l'élément optique (18) est interchangeable.

7. Support médical (200) destiné à une antisepsie par rayonnement ultraviolet - UV, comprenant une pluralité de dispositifs (100) selon l'une des revendications précédentes qui sont disposés en réseau, dans lequel les dispositifs (100) sont disposés sur un élément de support (110) flexible ou rigide.

8. Support médical (200) selon la revendication 7, dans lequel l'élément de support (110) comprend un élément d'espacement (112) flexible ou rigide, configuré pour ajuster une distance (A) entre les dispositifs (100) disposés en réseau et la surface (O) lorsqu'ils sont dans l'état appliqué sur une surface (O) à décoloniser, l'élément d'espacement (112) permettant une irradiation de la surface (O) avec le rayonnement émis par les dispositifs (100).

9. Support médical (200) selon la revendication 8, dans lequel l'élément d'espacement (112) est relié à l'élément de support (110) de manière interchangeable.

10. Support médical (200) selon l'une des revendications 7 à 9, dans lequel au moins une diode de surveillance (114) est intégrée au support (200) sous la forme d'un détecteur pour surveiller la puissance de rayonnement irradiée sur la surface (O) à décoloniser.
